# EUROPEAN PATENT APPLICATION

(11) **EP 4 400 102 A1**
(43) Date of publication of application: **17.07.2024**
(21) Application number: 24151471.0
(22) Date of filing: 11.01.2024
(51) Int. Cl.: A61K 31/198, A61K 33/14, A61K 33/30, A23L 33/16, A23L 33/175, A61P 13/00

(54) **PHARMACEUTICAL COMPOSITION AND METHOD FOR IMPROVING BIOMECHANICAL INTEGRITY OF THE PELVIC FLOOR**

(30) Priority: 12.01.2023 US 202363438705 P
(71) Applicant: Fempharma, LLC, 4029 Debrecen (HU)
(72) Inventor: TAKACS, Peter, H-4029 Debrecen (HU)
(74) Representative: D Young & Co LLP

(57) **Abstract**

Provided are a composition comprising creatine, leucine, zinc, calcium, and magnesium, formulated for oral delivery; a kit comprising the composition; and methods of treating urinary incontinence in a female subject by administering the composition to the subject.

## Description

### BACKGROUND

Approximately 50% of women will experience some form of urinary incontinence (UI) in their lifetime, as prevalence and age are positively correlated.³ UI is a significant economic burden, with annual cost estimates of $19.5 billion or more.⁴

Despite advancements in the management of stress UI, conservative treatment is recommended by most professional guidelines. The European Association of Urology (EAU) recommends that providers should offer supervised intensive pelvic floor muscle training (PFMT), lasting at least three months, as first-line therapy to all women with SUI or MUI (including elderly women) and to ensure that PFMT programs are as intensive as possible.⁵ The American College of Obstetricians and Gynecologists (ACOG) Practice Bulletin on UI states that behavioral modification and pelvic floor muscle exercises improve symptoms of UI and may be recommended as an initial, noninvasive treatment in any woman.⁶

A recent Cochrane systemic review compared PFMT with no treatment or inactive control treatment and found that women with stress UI in the PFMT groups were eight times more likely to report cure (56% vs. 6%).¹² However, a large percentage of women (44%) did not report a cure with PFMT alone. Therefore, there is a need for a regimen that results in improved treatment of UI.

### SUMMARY OF THE INVENTION

Some of the main aspects of the present invention are summarized below. Additional aspects are described in the Detailed Description of the Invention, Examples, Drawings, and Claims sections of this disclosure. The description in each section of this disclosure is intended to be read in conjunction with the other sections. Furthermore, the various embodiments described in each section of this disclosure can be combined in various different ways, and all combinations of disclosed embodiments are intended to fall within the scope of the present invention.

The present study demonstrates the impact of a specially formulated creatine-leucine-zinc-calcium-magnesium-containing composition on UI symptoms and improvement in pelvic floor muscle strength in women undergoing PFMT. This composition was designed to enhance the effect of resistance training on muscle strength and to increase physical performance in successive bursts of short-term, high-intensity exercise, which could help with stress-induced UI symptoms. This is the first report of the effects of a pharmaceutical composition comprising creatine, leucine, zinc, calcium, and magnesium on UI symptoms. This treatment regimen unexpectedly resulted in a twofold improvement in biomechanical integrity (BI) of the pelvic floor, compared with daily PFMT alone. The composition and methods of the invention improve UI symptoms, including stress urinary incontinence (SUI) symptoms, partly secondary to increased pelvic floor muscle strength.

Accordingly, in one aspect, the invention provides a pharmaceutical composition comprising 2-4 g creatine, 250-750 mg leucine, 2-8 mg zinc, 60-200 mg calcium, and 20-100 mg magnesium, wherein the composition is formulated for oral delivery. In a particular embodiment, the pharmaceutical composition comprises 3 g creatine, 500 mg leucine, 5 mg zinc, 120 mg calcium, and 60 mg magnesium.

Also provided is a kit comprising a pharmaceutical composition of the invention, optionally comprising instructions for administration of the pharmaceutical composition. In one embodiment the kit comprises instructions for PFMT.

In some embodiments, the pharmaceutical composition or the kit is for use in a method of improving BI of the pelvic floor of a female subject and/or for use in a method of treating or preventing urinary incontinence in a female subject.

The invention also provides a method of improving BI of the pelvic floor of a female subject undergoing PFMT. Other aspects include methods of treating or preventing urinary incontinence in a female subject undergoing PFMT.

In certain embodiments, the subject is elderly, is obese, has undergone pelvic surgery, has undergone radiation treatment in her pelvic area, has sustained traumatic injury to her pelvic area, or has sustained nerve damage in her pelvic area.

Methods of the invention comprise orally administering to the subject 2-4 g/day creatine, 250-750 mg/day leucine, 2-8 mg/day zinc, 60-200 mg/day calcium, and 20-100 mg/day magnesium, wherein administration continues daily for at least about six weeks. A specific embodiment comprises orally administering to the subject 3 g/day creatine, 500 mg/day leucine, 5 mg/day zinc, 120 mg/day calcium, and 60 mg/day magnesium daily for at least about six weeks. In some embodiments, administration is once daily.

PFMT comprises, in some embodiments, 30-60 pelvic floor muscle contractions per day. In a preferred embodiment, PFMT comprises 15 pelvic floor muscle contractions three time per day.

In certain embodiments, the subject's BI score is improved after at least about six weeks of treatment.

### BRIEF DESCRIPTION OF THE DRAWING

**FIG. 1** shows the flow of participants through the randomized clinical trial. Participants were allocated to the Treatment group or the Control group.
**FIG. 2A-2F** show boxplots for components of the Biomechanical Integrity (BI) Score of the Treatment group at baseline and at the completion of the study: Elasticity (**FIG. 2A**; P=0.035), Contraction (**FIG. 2B**), Support (**FIG. 2C**), Relaxation (**FIG. 2D**; P=0.0004), and Mobility (**FIG. 2E**). BI-score was significantly improved (**FIG. 2F**; P=0.0012).

### DETAILED DESCRIPTION OF THE INVENTION

Unless defined otherwise, all technical and scientific terms used herein have the same meaning as commonly understood by one of ordinary skill in the art to which this invention is related.

As used in this specification and the appended claims, the singular forms "a," "an," and "the" include plural referents, unless the context clearly dictates otherwise. The terms "a" (or "an") as well as the terms "one or more" and "at least one" can be used interchangeably.

Furthermore, "and/or" is to be taken as specific disclosure of each of the two specified features or components with or without the other. Thus, the term "and/or" as used in a phrase such as "A and/or B" is intended to include A and B, A or B, A (alone), and B (alone). Likewise, the term "and/or" as used in a phrase such as "A, B, and/or C" is intended to include A, B, and C; A, B, or C; A or B; A or C; B or C; A and B; A and C; B and C; A (alone); B (alone); and C (alone).

Units, prefixes, and symbols are denoted in their Système International de Unites (SI) accepted form. Numeric ranges are inclusive of the numbers defining the range. Where a numeric term is preceded by "about," the term includes the stated number and values ±10% of the stated number. The headings provided herein are not limitations of the various aspects or embodiments of the invention, which can be had by reference to the Specification as a whole. Accordingly, the terms defined immediately below are more fully defined by reference to the specification in its entirety.

Wherever embodiments are described with the language "comprising," otherwise analogous embodiments described in terms of "consisting of" and/or "consisting essentially of" are included.

An "effective amount" of an active agent or a pharmaceutical composition as disclosed herein is an amount sufficient to carry out a specifically stated purpose, in relation to the route of administration and dosage form.

An "active agent" is an agent which itself has biological activity, or which is a precursor or prodrug that is converted in the body to an agent having biological activity.

The term "pharmaceutical composition" refers to a collection of ingredients or substituents in such form as to permit the biological activity of the active ingredient(s) to be effective, and which contains no additional components that are unacceptably toxic to a subject to which the composition would be administered. In embodiments in which more than one active agent is administered, the agents can be administered together (for example, in the same formulation and/or at the same time), or separately (for example, in different formulations and/or at different times). Accordingly, a pharmaceutical composition of the invention can comprise one or multiple dosage units.

Pharmaceutical compositions generally can be in numerous dosage forms or combinations of dosage forms. Dosage forms for oral delivery include, for example, tablet, capsule, liquid, solution, softgel, suspension, emulsion, syrup, elixir, tincture, film, powder, and/or hydrogel. "Oral delivery" includes, without limitation, oral, sublingual, and buccal delivery.

Provided herein is a pharmaceutical composition formulated for oral delivery comprising creatine, leucine, zinc, calcium, and magnesium. The present study indicates that the unique composition of the invention, containing these key ingredients in a unique ratio, is likely responsible for the observed beneficial effects on pelvic floor muscle strength, better BI score, and improvement in symptoms of UI of in subjects receiving the composition.

Creatine is a non-essential nitrogenous organic acid that occurs in vertebrates, and it is also synthesized in the human body from L-arginine, glycine, and L-methionine. Creatine is the primary constituent of phosphocreatine, which is used to regenerate ATP within the cell. Approximately 95 % of the creatine pool in the body is located in skeletal muscle. About 1-2% of intramuscular creatine is degraded into creatinine and excreted in the urine. ¹³⁻¹⁵ Therefore, the body needs to replenish about 1-3 g of creatine per day to maintain normal creatine stores depending on muscle mass. About half of the daily need for creatine is obtained from the diet. ^{16,17}

The European Food Safety Authority (EFSA) has approved several health claims for creatine, including that daily creatine consumption can enhance the effect of resistance training on muscle strength in adults over the age of 55 and that creatine increases physical performance in successive bursts of short-term, high-intensity exercise.¹⁸ However, the influence of creatine on the BI of the pelvic floor has not been previously demonstrated.

In some embodiments, the pharmaceutical composition of the invention comprises a total of about 1-5 g, a total of at least about 1, 1.5, 2, 2.5, 3, 3.5, 4, 4.5, or 5 g creatine, or any dosage range of creatine having these amounts as endpoints. In one embodiment, the amount of creatine is about 3 g. The creatine can be in any suitable form, including, for example, anhydrate, monohydrate, ester, and salt forms, such as citrate, magnesium, malate, orotate, phosphate, or pyruvate. In a particular embodiment, the composition comprises creatine monohydrate. In one embodiment, the pharmaceutical composition of the invention comprises a total of about 1-5 g of creatine monohydrate. In one embodiment, the pharmaceutical composition of the invention comprises or a total of at least about 1, 1.5, 2, 2.5, 3, 3.5, 4, 4.5, or 5 g creatine monohydrate.

Leucine is an essential amino acid, capable of stimulating the synthesis of muscle proteins. It enhances regeneration and prevents muscle tissue from degradation. Leucine can also prevent muscular weakness related to aging.¹⁹ In addition, several research studies have shown leucine's beneficial role in enhancing muscle protein synthesis.¹⁹⁻²¹ For example, postpartum women receiving a specially formulated leucine-containing postpartum recovery composition had improved recovery of the pelvic floor after vaginal delivery.²²

The pharmaceutical composition of the invention can comprise a total of about 100 mg to 1 g leucine, a total of at least about 100, 150, 200, 250, 300, 350, 400, 450, 500, 550, 600, 650, 700, 750, 800, 850, 900, 950, or 1,000 mg leucine, or any dosage range of leucine having these amounts as endpoints. In one embodiment, the amount of leucine is about 500 mg.

Zinc is one of the most important trace elements in the human body; it is a cofactor for many enzymes and also acts as a structural, catalytic, or signal mediator in nearly 10 % of human proteins. It is essential in wound healing, connective tissue biosynthesis, and homeostasis.³⁸ Zinc levels have been shown to decline with age³⁹, and therapeutic interventions aiming to treat vaginal atrophy have been shown to increase the zinc levels in the vagina.⁴⁰

The pharmaceutical composition of the invention can comprise at total of about 2-8 mg of zinc, a total of at least about 2, 3, 4, 5, 6, 7, or 8 mg zinc, or any dosage range of zinc having these amounts as endpoints. In one embodiment, the amount of zinc is about 5 mg. The zinc can be in any suitable form including, for example, as acetate, citrate, gluconate, orotate, picolinate, or sulfate. In a particular embodiment, the composition comprises a zinc sulfate, such as zinc sulfate heptahydrate. In one embodiment, the pharmaceutical composition of the invention comprises a total of about 2-8 mg of zinc sulfate. In one embodiment, the pharmaceutical composition of the invention comprises a total of at least about 2, 3, 4, 5, 6, 7, or 8 mg zinc sulfate. In one embodiment, the pharmaceutical composition of the invention comprises a total of about 2-8 mg of zinc sulfate heptahydrate. In one embodiment, the pharmaceutical composition of the invention comprises a total of at least about 2, 3, 4, 5, 6, 7, or 8 mg zinc sulfate heptahydrate.

Calcium and magnesium are critical elements in muscle function to maintain physical fitness.⁴¹ Altered calcium homeostasis, as frequently found in the elderly, together with oxidative stress, is implicated in augmented muscle damage, decreased muscle mass, and impaired muscle contractility.⁴¹ On the other hand, magnesium is involved in protein and ATP synthesis and responsible for muscle relaxation⁴¹ A randomized clinical trial revealed that elderly women receiving daily supplements with magnesium for 12 weeks in addition to an exercise program, had improved physical performance, suggesting a role for magnesium supplementation in maintaining muscle function and helping to delay sarcopenia.⁴² The EFSA has approved several health claims regarding magnesium and calcium: both contribute to normal muscle function, while magnesium contributes to a reduction of tiredness and fatigue, and calcium contributes to normal neurotransmission.

Pharmaceutical compositions of the invention can comprise a total of about 60-200 mg calcium, a total of at least about 60, 65, 70, 75, 80, 85, 90, 95, 100, 110, 120, 130, 140, 150, 160, 170, 180, 190, or 200 mg calcium, or any dosage range of calcium having these amounts as endpoints. In one embodiment, the amount of calcium is about 120 mg. The calcium can be in any suitable form including, for example, as carbonate or citrate. In a particular embodiment, the composition comprises calcium citrate. In one embodiment, the pharmaceutical composition of the invention comprises a total of about 60-200 mg calcium citrate. In one embodiment, the pharmaceutical composition of the invention comprises a total of at least about 60, 65, 70, 75, 80, 85, 90, 95, 100, 110, 120, 130, 140, 150, 160, 170, 180, 190, or 200 mg calcium citrate.

In some embodiments, the pharmaceutical composition of the invention comprises a total of about 20-100 mg magnesium, a total of at least about 20, 25, 30, 35, 40, 45, 50, 55, 60, 65, 70, 75, 80, 85, 90, 95, or 100 mg magnesium, or any dosage range of magnesium having these amounts as endpoints. In one embodiment, the amount of magnesium is 60 mg. The magnesium can be in any suitable form including, for example, as carbonate, chloride, citrate, glycinate, lactate, malate, or oxide. In a particular embodiment, the composition comprises magnesium citrate. In one embodiment, the pharmaceutical composition of the invention comprises a total of about 20-100 mg magnesium citrate. In one embodiment, the pharmaceutical composition of the invention comprises a total of at least about 20, 25, 30, 35, 40, 45, 50, 55, 60, 65, 70, 75, 80, 85, 90, 95, or 100 mg magnesium citrate.

By "subject" or "individual" or "patient" is meant a mammalian subject, for whom diagnosis, prognosis, therapy, or prevention is desired. In one embodiment, the subject is a human.

The subject may suffer from or be at risk of developing urinary incontinence (UI), characterized by involuntary urine leakage. Stress urinary incontinence (SUI) is the complaint of involuntary leakage on effort or exertion or on sneezing or coughing, while mixed urinary incontinence is the complaint of involuntary leakage associated with urgency and also with exertion, effort, sneezing, or coughing.¹ Mixed urinary incontinence (MUI) is regarded as stress-predominant when stress incontinence episodes dominate.²

In some embodiments, the invention provides methods of treating UI in a subject or of preventing UI in a subject susceptible to developing UI, wherein the subject is undergoing PFMT. PFMT can comprise, for example, performance by the subject of 30-60 pelvic floor muscle contractions per day. The pelvic floor muscle contractions can be performed at least once, twice, three, or four times per day. For example, the subject can perform 10 contractions 3, 4, 5, or 6 times per day; or 15 contractions 2, 3, or 4 times per day; or 20 contractions 2 or 3 times per day. PFMT can comprise electrical stimulation of the pelvic floor.

Terms such as "treating" or "treatment" or "to treat" or "alleviating" or "to alleviate" refer to therapeutic measures that cure, slow down, lessen symptoms of, and/or halt progression of a diagnosed pathologic condition or disorder. Thus, those in need of treatment include those already with the disorder. In certain embodiments, a subject is successfully "treated" for a disease or disorder according to the methods provided herein if the patient shows, *e.g*., total, partial, or transient alleviation or elimination of symptoms associated with the disease or disorder; diminishment of the extent of the condition, disease, or disorder; stabilization (*i.e*., not worsening) of the condition, disease, or disorder; or slowing of progression of the condition, disease, or disorder.

"Prevent" or "prevention" refers to prophylactic or preventative measures that prevent and/or slow the development of a targeted pathologic condition or disorder. Thus, those in need of prevention include those at risk of or susceptible to developing the disorder. Subjects that are at risk of or susceptible to developing UI include, but are not limited to, women who are elderly (*i.e.,* aged 65 or older), women who are obese, women who have undergone pelvic surgery or radiation treatment in the pelvic area, women who have sustained a traumatic injury and/or nerve damage in the pelvic area. In certain embodiments, a disease or disorder is successfully prevented according to the methods provided herein if the patient develops, transiently or permanently, *e.g*., fewer or less severe symptoms associated with the disease or disorder, or a later onset of symptoms associated with the disease or disorder, than a patient who has not been subject to the methods of the invention.

UI can be assessed using a variety of measures.

For example, the Medical, Epidemiologic, and Social Aspects of Aging (MESA) questionnaire is a reliable and validated tool developed and validated to identify the urgency- or stress-predominant component of MUI and assess the severity of symptoms.^{23,24} The MESA questionnaire consists of two separate parts: nine questions regarding SUI and six questions concerning urgency urinary incontinence (UUI). The maximum total score for SUI is twenty-seven, while eighteen for UUI, with 0 to 3 allocated to the answers of never/rarely/sometimes/often. The stress/urge index is calculated to detect stress- or urge-predominant MUI. The indices are obtained by dividing the actual score of each category by the maximum total possible. The MUI is categorized as stress-predominant when the stress index is greater than the urge index. To assess the severity of stress incontinence, the total score of stress (maximum = 27) is divided into three third categories. For the stress category, scores of 1 to 9 are assigned as mild, 10 to 18 as moderate, and 13 to 18 as severe.

Urinary Distress Inventory (UDI-6) consists of six items and is a short version of a condition-specific quality-of-life instrument.^{28,29} To specify the severity of urinary distress symptoms, patients' response options ranged from 0 ("no symptoms") to 4 ("quite a bit"). To calculate the score, the mean score of answered items within each component is multiplied by 25 to obtain the scale score (range 0-100). Higher scores in UDI-6 indicate a higher disability.

Incontinence Impact Questionnaire (IIQ-7) is a urinary incontinence-specific psychometric questionnaire. This questionnaire consists of 7 items, and assesses the psychosocial impact of UI in women. The total score ranges from 0 to 100.^{28,29} Higher score indicates a worse quality of life.

Assessment of pelvic floor strength during gynecological examination may help to identify women at risk of UI. Pelvic floor muscle function can be assessed in a variety of ways. For example, vaginal squeeze pressure has been used as a marker for pelvic floor function.³⁴ Pelvic floor muscle contraction can be measured with palpation, perineometry, and by ultrasound. Magnetic resonance imaging (MRI) and measurement using a dynamometer are other methods of assessing pelvic floor muscle function. A perineometer or vaginal manometer is a very effective and precise instrument for measuring the strength of voluntary contractions of the pelvic floor muscles. The handheld clinical biofeedback perineometer is controlled by a microprocessor. Pelvic floor contraction causes air pressure in the sensor to be transferred through the connecting tube and displayed on the readout unit.

One embodiment of the invention comprises a method of improving BI of the pelvic floor of a female subject undergoing PFMT.

A Vaginal Tactile Imager (VTI) can be used to assess BI of the pelvic floor. A VTI examination consists of eight Tests as follows: 1) probe insertion, 2) elevation, 3) rotation, 4) Valsalva maneuver, 5) voluntary muscle contraction (anterior versus posterior compartments), 6) voluntary muscle contraction (left versus right side), 7) involuntary relaxation, and 8) reflex muscle contraction (cough). Tests 1, 2, 4, 5, 7, and 8 provide data for anterior/posterior compartments; Test 3 provides data for 360 degrees; Test 6 provides data for left/right sides. The VTI probe allows 3-15 mm tissue deformation at the probe insertion (Test 1), 20-45 mm tissue deformation at the probe elevation (Test 2), and 5-7 mm deformation at the probe rotation (Test 3) and recording of dynamic responses at Valsalva maneuver, pelvic muscle contractions, and relaxation (Tests 4-8). The probe maneuvers in Tests 1-3 are used to accumulate multiple pressure patterns from the tissue surface and create an integrated tactile image for the investigated area employing the image composition algorithms. The spatial gradients ∂P(x, y)/∂y for anterior and posterior compartments are calculated within the acquired tactile images in Test 1 and 2; the y-coordinate is directed orthogonally from the vaginal channel coming through anterior-posterior compartments, and the x-coordinate is located on the vaginal channel. Software automatically calculates 52 VTI parameters for eight test procedures.

BI-score covers biomechanical aspects of the pelvic floor, which include tissue elasticity, pelvic support, muscle contraction, involuntary relaxation, and mobility.²⁷ In certain embodiments, BI of the pelvic floor of a subject undergoing PFMT and administered a composition comprising creatine, leucine, zinc, calcium, and magnesium is improved when a subject's BI score is increased after at least about six weeks of treatment. Subjects' BI scores can be improved, for example, by an average of at least about 0.40 (SD), 0.41 (SD), 0.42 (SD), 0.43 (SD), 0.44 (SD), 0.45 (SD), 0.46 (SD), 0.47 (SD), 0.48 (SD), 0.49 (SD), 0.50 (SD), 0.51 (SD), 0.52 (SD), 0.53 (SD), 0.54 (SD), 0.55 (SD), 0.56 (SD), 0.57 (SD), 0.58 (SD), 0.59 (SD), or 0.60 (SD), compared to average baseline BI score. Subjects' BI scores can be improved, for example, by an average of at least about 40%, 41%, 42%, 43%, 44%, 45%, 46%, 47%, 48%, 49%, 50%, 51%, 52%, 53%, 54%, or 55%, compared to average baseline BI score.

In some embodiments, a subject's BI score is improved by at least about 0.40 (SD), 0.41 (SD), 0.42 (SD), 0.43 (SD), 0.44 (SD), 0.45 (SD), 0.46 (SD), 0.47 (SD), 0.48 (SD), 0.49 (SD), 0.50 (SD), 0.51 (SD), 0.52 (SD), 0.53 (SD), 0.54 (SD), 0.55 (SD), 0.56 (SD), 0.57 (SD), 0.58 (SD), 0.59 (SD), or 0.60 (SD), compared to the subject's baseline BI score. In some embodiments, a subject's BI score is improved by at least about 40%, 41%, 42%, 43%, 44%, 45%, 46%, 47%, 48%, 49%, 50%, 51%, 52%, 53%, 54%, or 55%, compared to the subject's baseline BI score. In particular embodiments, the subject's BI score is improved by at least about 0.49 (SD) or by at least about 46%, compared to the subject's baseline BI score.

Patients subjected to methods of the present invention can demonstrate one or more of improved maximum vaginal squeeze pressure, improved PGI-S score, improved UDI-6 score, improved IIQ7 score, and/or improved BI score. Improvement is measured after at least 6 weeks of a daily treatment regimen comprising PFMT and oral administration of 2-4 g creatine, 250-750 mg leucine, 2-8 mg zinc, 60-200 mg calcium, and 20-100 mg magnesium. Improvement is relative to the subject's baseline parameters, as measured prior to daily administration of creatine, leucine, zinc, calcium, and magnesium.

Alternatively, improvement can be assessed relative to a control population. For example, when comparing the vaginal squeeze pressure of a subject undergoing PFMT and receiving the pharmaceutical composition of the invention for at least six weeks, that subject's vaginal squeeze pressure is compared with the average vaginal squeeze pressure of subjects undergoing PFMT for a comparable time period, who did not receive the pharmaceutical composition of the invention.

The pharmaceutical composition can be administered according to any suitable dosing regimen, for example, where the daily dose is divided into two or more separate doses. It is within the skill of the ordinary artisan to determine a dosing schedule and duration. In some embodiments, the pharmaceutical composition is administered orally once a day or in a divided dose twice a day. Components of the pharmaceutical composition can be administered as a single dosage form or as multiple dosage forms, each comprising one or more components of the composition.

Preferably, 2-4 g/day creatine, 250-750 mg/day leucine, 2-8 mg/day zinc, 60-200 mg/day calcium, and 20-100 mg/day magnesium are administered to a subject undergoing PFMT for at least about 6, 7, 8, 9, 10, 11, or 12 weeks. Typically, administration is self-administration.

Also within the scope of the invention are kits comprising the pharmaceutical compositions as provided herein and instructions for use. Kits typically include a label indicating the intended use of the contents of the kit. The term "label" includes any writing, or recorded material supplied on or with the kit, or which otherwise accompanies the kit. The label may include, for example, instructions for administration. In one embodiment, the kit includes instructions for PFMT.

A kit of the invention generally comprises multiple doses of the pharmaceutical composition, for example, 7, 14, 21, 28, 35, or 42 daily doses of the pharmaceutical composition of the invention. In some embodiments, the components of each daily dose are packaged together, for example, in a pouch, blister pack, etc. In some embodiments, the components of each daily dose are divided into two packages for administration twice daily.

All of the references cited in this disclosure are hereby incorporated by reference in their entireties. In addition, any manufacturers' instructions or catalogues for any products cited or mentioned herein are incorporated by reference. Documents incorporated by reference into this text, or any teachings therein, can be used in the practice of the present invention. Documents incorporated by reference into this text are not admitted to be prior art.

Embodiments of the present disclosure can be further defined by reference to the following non-limiting examples. It will be apparent to those skilled in the art that many modifications, both to materials and methods, can be practiced without departing from the scope of the present disclosure.

### EXAMPLES

### Example 1. Treatment of Urinary Incontinence

### Study Design and Enrollment

A randomized, double-blind, placebo-controlled trial with a six-week follow-up period was conducted to assess the effectiveness of a specially formulated creatine-leucine-zinc-calcium-magnesium-based composition and PFMT in women with stress-predominant urinary incontinence.

The randomization was done in a 1:1 ratio and generated by SAS (SAS Institute, Cary, NC) version 9.4. Women with stress or stress-predominant urinary incontinence, based on the MESA questionnaire, were enrolled.^{23,24} Women who indicated stress UI or stress-dominant mixed UI (stress percent score more than urge percent score) were eligible for inclusion. Exclusion criteria were pregnancy or less than 12 months postpartum; more than three vaginal deliveries or any prior operative delivery; self-reported symptoms of pelvic organ prolapse or POP-Q stage >2; history of supervised PFMT within 12 months; and current medications for UI or surgical treatment for UI; known zinc or copper deficiency or sensitivity; collagen or connective tissue disease.

After enrollment, women were randomized to receive daily oral administration for six weeks with either a specially formulated composition (creatine monohydrate 3g/day, leucine 0.5g/day, zinc sulfate heptahydrate 5mg/day, calcium citrate 120mg/day and magnesium citrate 60mg/day) (treatment group) or a placebo (maltodextrin) (control group). Patients and evaluators were blinded to the allocation.

Patients received detailed pelvic floor muscle exercise instruction and training with the aid of the perineometer. Study participants were instructed to perform daily pelvic floor muscle exercises at an intensity of at least 65-75% of one repetition maximum of 45 pelvic floor muscle (PFM) contractions per day (15 PFM contractions performed three times per day) for six weeks as per previous protocols.^{7,8} This PFMT volume has been shown to be manageable with regard to exercise adherence and improved UI symptoms.^{10,11}

The objective measurements of pelvic floor integrity by VTI were complemented by subjective patient assessments based on validated questionaries. In particular, patients completed the UDI-6 and IIQ-7 assessments, and underwent a standardized pelvic exam, Pelvic Organ Prolapse Quantification (POP-Q) measurement, Oxford scale assessment of pelvic floor strength, measurement by a perineometer device (Peritron, Laborie, Williston, Vermont) of vaginal squeeze pressure,^{25,26} and evaluation by Vaginal Tactile Imager (VTI) of the biomechanical integrity of the pelvic floor.²⁷ Evaluators performing the perinoemetry, VTI, and pelvic floor assessment were blinded to the allocation of subjects in the control or treatment group.

VTI, model 2S, was used for biomechanical mapping of the pelvic floor. The VTI probe has 96 pressure (tactile) sensors spaced consecutively on both sides of the probe, an orientation sensor, and temperature controllers to provide the probe temperature close to a human body before the examination. During the clinical procedure, the probe is used to acquire pressure responses from two opposite vaginal walls along the vagina. The tactile images of the vagina integrate all the acquired pressure and positioning data for each pressure-sensing element during vaginal wall deformation and pelvic muscle contraction. The VTI software calculates (automatically) 52 VTI parameters for eight test procedures listed in **Table 3**.

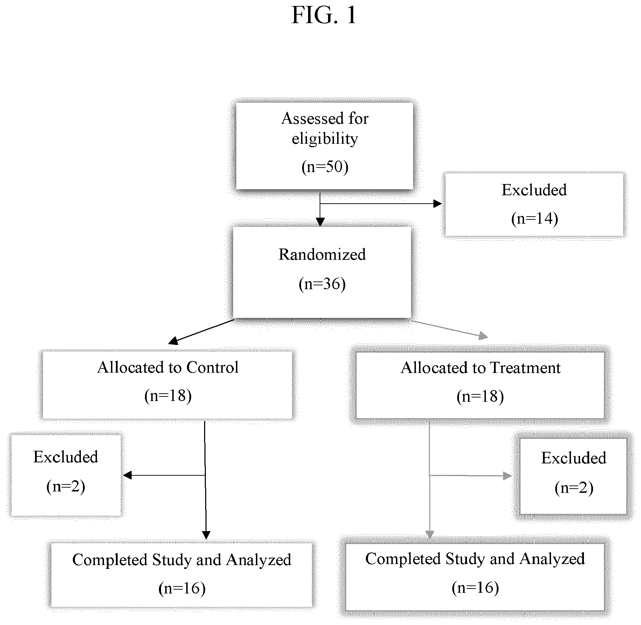

**FIG. 1** summarizes the assessment for eligibility, randomization, allocation, and exclusions of participants. Of the fifty women screened, thirty-six met the entry criteria and were randomized. Thirty-two women completed the trial. Of the fourteen excluded participants, ten did not meet the inclusion criteria, four decided not to participate in the trial. Eighteen women were randomly assigned to the control group and eighteen to the treatment group.

Of the eighteen individuals randomly assigned to the treatment group, two did not complete the trial: one was lost to follow-up, and one withdrew from participation because of lack of time. Of the eighteen women randomized to the control group, two did not complete the trial: one was lost to follow-up, and one withdrew consent before taking any supplement. In total, thirty-six women completed the trial: sixteen in the control group and sixteen in the treatment group. There were no demographic differences between the two groups (**Table 1**).

At the six-week visit, study participants were asked to complete the UDI-6 and IIQ-7 questionnaires again. In addition, repeat pelvic exams, including POP-Q measurement, Oxford scale assessment of the pelvic floor strength, the strength of voluntary contractions of vaginal muscles was measured by a perineometer device in a standardized fashion, and VTI evaluated the BI of the pelvic floor. Participants self-reported high compliance (~90%) in adhering to the daily treatment regimen of the pharmaceutical composition (or placebo) and PFMT.

The primary outcome was the UDI-6 score. Secondary outcomes were the IIQ-7 score, PGI-S and PGI-I, Biomechanical Integrity score (BI-score) measured by VTI, and vaginal squeeze pressure measured by perineometer.

PGI-S is a global index that may be used to rate the severity of a specific condition (a single-state scale). PGI-S was validated on women with SUI. ³⁰ PGI-S, range 0 = no symptoms to 4 = severe symptoms, MID = 1.³¹ PGI-I is a seven-point transition scale that comprises a single question asking patients to rate their urinary tract condition now as compared with how it was before treatment on a scale from 1 (very much better) to 7 (very much worse).³⁰

### Results

MESA SUI scores were similar between the control and treatment groups (mean ± SD, 15.3±5.5 vs. 13.2±5.0; P=0.25). Between-group analysis revealed no significant differences between the control and treatment group except for mean change (increase) in vaginal squeeze pressure [(cmH2O, mean ± SD), 5±12 vs. 15±15, P=0.04] and mean change (decrease) in PGI-S score [(mean ± SD), -0.2±0.9 vs. -0.8±0.8, P=0.04] (**Table 2**). Out of the 52 examined VTI parameters, significantly more parameters improved in the treatment group compared to the control group (11/52 vs. 3/52, P=0.04) (**Table 3** and **Table 4**).

Within-group analysis showed that UDI-6 and IIQ-7 scores improved significantly from baseline to six weeks in the treatment group but not in the control group [UDI-6 score (mean ± SD) 45±21 vs. 29±21, P=0.02 (treatment group), 43±18 vs. 33±26, P=0.22 (control group)] [IIQ-7 score before (mean ± SD) 50±30 vs. 30±21, P=0.01 (treatment group) 48±23 vs.40±28, P=0.36] (**Table 5**). Similarly, maximum vaginal squeeze pressure was significantly stronger in the treatment group compared to baseline after six weeks but not in the control group [Maximum vaginal squeeze pressure (cmH2O, mean ± SD), 30±15 vs. 45±28, P=0.001 (treatment group) and 36±18 vs. 41±21, P=0.13 (control group)]. PGI-S scores improved only in the treatment group from baseline to six weeks after treatment [PGI-S score (mean ± SD) 3.1±0.8 vs. 2.3±0.8, P=0.0001]. The increase in vaginal squeeze pressure in the treatment group, likely contributing to the improved PGI-S change.

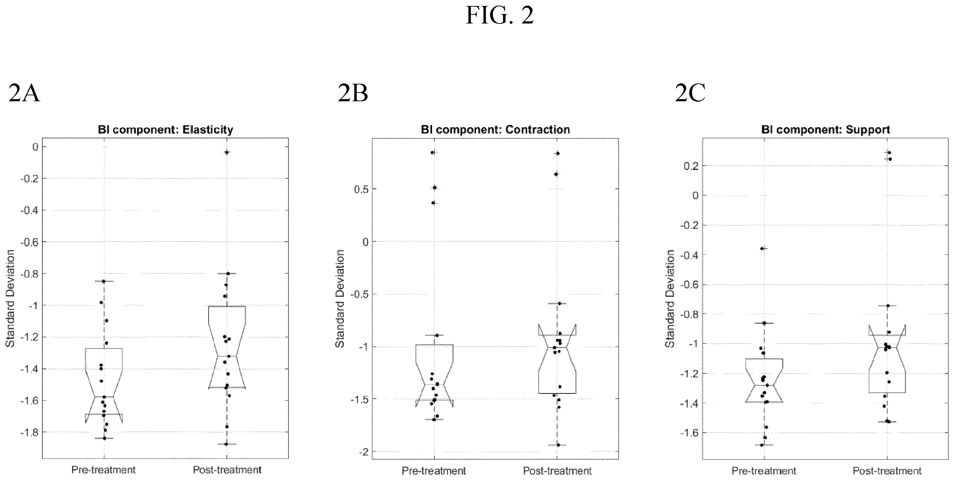

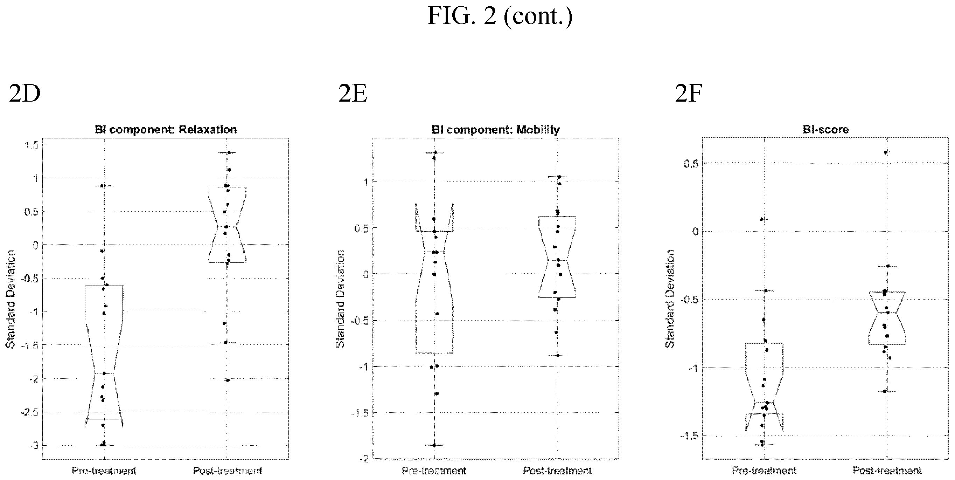

VTI revealed that in the control group, only three VTI parameters improved (VTI #6, 10, 49), while in the treatment group, eleven parameters were better (VTI #1,5, 19, 37, 40-46) (**Table 3** and **Table 4**). None of the VTI parameters worsened during the trial (**Table 3** and **Table 4**). BI-score, on average, improved in the treatment group (**FIG. 2A-2F**) and the control group [SD unit, mean, from -1.06 to -0.58, P=0.001 (treatment group) and from -0.66 to -0.42, P=0.04 (control group)] (**Table 6** and **Table 7**). However, women in the treatment, on average, improved twice as much in their BI-score as women in the control group (-0.49 vs. -0.24). There was a significant improvement in elasticity and relaxation in the treatment group, while in the control group, only improvement in contraction was observed (**Table 6** and **Table 7**).

### Statistical Analysis

SigmaStat/SPSS software was used for statistical calculations. Descriptive statistics were calculated for all variables of interest. Means and standard deviations were calculated for continuous outcomes. Frequency and percentage were calculated for categorical outcomes. The Fisher exact test was used to compare frequencies. Student's t-test was used to compare the mean values between the two groups. Paired t-test was used to compare paired data, which was obtained at baseline and six weeks. Wilcoxon signed rank test was used to compare the VTI data.

The study had adequate power to detect a difference after 32 patients' enrollment. Power analysis was performed [G*Power Statistical Power Analyses Software³²] based on a pilot study, which revealed that in our population of women with SUI, the baseline UDI score was 36±15. To have a power of 80% and a significance level of 5% with a large effect size (Cohen's d=1.06) to detect a decrease of 16 points in the UDI-6 score, a sample size of 32 was needed with 16 patients in each arm of the trial. The MCID for the UDI-6 questionnaire is 11 points.³³ Calculating with a dropout rate of 10%, thirty-four women were needed to be enrolled.

Statistical significance was defined as a P-value <0.05 using two-tailed tests.

### Tables

**Table 1. Demographics and Clinical Characteristics**

| | | **Control Group (N=16)** | **Treatment Group (N=16)** | **P-value** |
|---|---|---|---|---|
| Age (years, mean ± SD | | 57±12 | 53±10 | 0.28 |
| Gravida (median, range) | | 2 (1-4) | 2 (1-3) | NS |
| Parity (median, range) | | 2 (1-3) | 2 (1-5) | NS |
| BMI (kg/m², mean ± SD) | | 26.6±5.3 | 28.6±5.9 | 0.32 |
| History of vaginal delivery (n, %) | | 14 (87) | 16 (100) | NS |
| Postmenopausal (n, %) | | 9 (56) | 8 (50) | NS |
| POP-Q stage (median, range) | | 2 (1-2) | 2 (1-2) | NS |
| MESA SUI index (%, mean ± SD) | | 57±20 | 49±19 | 0.25 |
| MESA UUI index (%, mean ± SD) | | 13±14 | 17±13 | 0.46 |
| MESA SUI score (mean ± SD) | | 15.3±5.5 | 13.2±5.0 | 0.25 |
| Severity of SUI | | | | |
| | Mild (n, %) | 3 (19) | 5 (31) | |
| | Moderate (n, %) | 8 (50) | 8 (50) | |
| | Severe (n, %) | 5 (31) | 3 (19) | |

| | | | | |
|---|---|---|---|---|
| NS: not significant, POP-Q: pelvic organ prolapse quantification, SUI: stress urinary incontinence, UUI: urge urinary incontinence, MESA: Medical, epidemiologic, and social aspects of aging urinary incontinence questionnaire, Stress urinary incontinence severity category: scores of 1 to 9 is assigned as mild, 10 to 18 as moderate, and 19 to 27 as severe. | | | | |

**Table 2. Between-Group Analysis of Primary and Secondary Outcomes at Baseline and Study Completion**

| | **Control Group (N=16)** | **Treatment Group (N=16)** | **P-value** |
|---|---|---|---|
| UDI-6 score before (mean ± SD) | 43±18 | 45±21 | 0.77 |
| UDI-6 score after (mean ± SD) | 33±26 | 29±22 | 0.69 |
| UDI-6 score change (mean ± SD) | -10±31 | -16±25 | 0.58 |
| IIQ-7 score before (mean ± SD) | 48±23 | 50±30 | 0.76 |
| IIQ-7 score after (mean ± SD) | 40±28 | 30±21 | 0.31 |
| IIQ-7 score change (mean ± SD) | -8±30 | -20±27 | 0.25 |
| Maximum vaginal squeeze pressure before (cmH₂O, mean ± SD) | 36±18 | 30±15 | 0.27 |
| Maximum vaginal squeeze pressure after (cmH₂O) | 41±21 | 45±28 | 0.71 |
| Change in vaginal squeeze pressure (cmH₂O, mean ± SD) | 5±12 | 15±15 | **0.04** |
| Oxford Scale before (mean ± SD) | 2.4±0.8 | 2.2±0.6 | 0.51 |
| Oxford Scale after (mean ± SD) | 2.9±0.5 | 2.8±0.5 | 0.53 |
| Oxford Scale change (mean ± SD) | 0.5±0.7 | 0.6±0.5 | 0.78 |
| PGI-S score before (mean ± SD) | 2.8±0.8 | 3.1±0.8 | 0.31 |
| PGI-S score after (mean ± SD) | 2.6±0.8 | 2.3±0.8 | 0.19 |
| PGI-S score change (mean ± SD) | -0.2±0.9 | -0.8±0.8 | **0.04** |
| PGI-I score (mean ± SD) | 2.4±1.0 | 2.6±0.9 | 0.61 |

| | | | |
|---|---|---|---|
| Urogenital Distress Inventory (UDI-6), Incontinence Impact Questionnaire (IIQ-7), Patient Global Impression of Severity and Improvement question (PGI-S and PGI-I) | | | |

**Table 3. VTI Parameters for Control Group at Baseline and Study Completion**

| **VTI Parameter No.** | **Parameter Units** | **Average (Baseline) (N=16)** | **Average (6 weeks) (N=16)** | **100* (Post-Pre)/Pre, %** | **PreTreatment Std. Dev.** | **PostTreatment Std. Dev.** | **P-value for Wilcoxon Signed Rank Test** |
|---|---|---|---|---|---|---|---|
| 1 | N | 0.55 | 0.67 | 20.8 | 0.42 | 0.40 | 0.4545 |
| 2 | mJ | 21.38 | 26.49 | 23.9 | 13.84 | 17.09 | 0.0768 |
| 3 | kPa/mm | 0.83 | 1.04 | 25.0 | 0.67 | 0.81 | 0.3018 |
| 4 | kPa/mm | 0.64 | 0.80 | 25.8 | 0.49 | 0.53 | 0.4545 |
| 5 | kPa | 14.38 | 16.49 | 14.7 | 11.47 | 10.98 | 0.4545 |
| 6 | kPa | 9.13 | 11.76 | 28.8 | 5.00 | 6.69 | 0.0042 |
| 7 | kPa | 7.69 | 9.04 | 17.5 | 7.54 | 9.06 | 0.8036 |
| 8 | kPa | 5.44 | 6.13 | 12.6 | 3.42 | 4.46 | 1.0000 |
| 9 | kPa | 4.40 | 5.39 | 22.4 | 3.09 | 3.08 | 0.8036 |
| 10 | kPa | 5.58 | 7.13 | 27.8 | 3.72 | 4.35 | 0.0213 |
| 11 | kPa | 6.51 | 6.19 | -4.9 | 3.56 | 3.50 | 1.0000 |
| 12 | kPa | 4.83 | 4.57 | -5.3 | 2.95 | 3.88 | 0.6072 |
| 13 | kPa/mm | 0.69 | 0.58 | -15.1 | 1.18 | 1.15 | 1.0000 |
| 14 | kPa/mm | 0.33 | 0.31 | -5.7 | 0.41 | 0.31 | 1.0000 |
| 15 | kPa/mm | 0.20 | 0.26 | 26.2 | 0.18 | 0.18 | 0.4545 |
| 16 | kPa/mm | 0.60 | 0.47 | -22.1 | 1.03 | 0.30 | 0.8036 |
| 17 | kPa/mm | 0.34 | 0.25 | -25.8 | 0.46 | 0.14 | 0.8036 |
| 18 | kPa/mm | 0.23 | 0.19 | -14.7 | 0.19 | 0.17 | 1.0000 |
| 19 | kPa | 11.95 | 15.59 | 30.4 | 8.47 | 9.92 | 0.2101 |
| 20 | N | 1.91 | 2.45 | 28.2 | 0.88 | 1.24 | 0.4545 |
| 21 | N | 0.98 | 1.17 | 18.9 | 0.51 | 0.79 | 0.6072 |
| 22 | kPa | 3.95 | 4.74 | 20.1 | 1.75 | 2.68 | 0.2101 |
| 23 | kPa | 4.05 | 4.38 | 8.2 | 2.34 | 3.47 | 0.4545 |
| 24 | kPa | 4.59 | 5.68 | 23.8 | 2.38 | 3.42 | 1.0000 |
| 25 | N | 0.99 | 0.96 | -2.6 | 0.72 | 0.48 | 1.0000 |
| 26 | kPa | 7.25 | 5.31 | -26.7 | 8.17 | 3.18 | 0.8036 |
| 27 | mm | 1.27 | 2.44 | 92.6 | 6.90 | 5.38 | 1.0000 |
| 28 | N | 0.96 | 1.09 | 13.8 | 0.66 | 0.59 | 0.8036 |
| 29 | kPa | 4.90 | 5.40 | 10.2 | 3.33 | 3.88 | 1.0000 |
| 30 | mm | -0.08 | 2.28 | -2900.0 | 4.04 | 6.10 | 0.8036 |
| 31 | N | 1.17 | 1.50 | 27.8 | 0.83 | 0.95 | 0.8036 |
| 32 | kPa | 17.55 | 21.74 | 23.9 | 16.65 | 17.57 | 1.0000 |
| 33 | kPa | 23.21 | 27.67 | 19.2 | 17.84 | 19.87 | 0.4545 |
| 34 | N | 1.25 | 1.68 | 35.2 | 0.65 | 0.78 | 0.4545 |
| 35 | kPa | 9.49 | 12.01 | 26.5 | 6.09 | 6.54 | 0.2101 |
| 36 | kPa | 14.54 | 17.81 | 22.4 | 7.36 | 9.19 | 0.0768 |
| 37 | N | 0.58 | 0.79 | 36.8 | 0.39 | 0.55 | 0.4545 |
| 38 | kPa | 4.73 | 5.59 | 18.2 | 3.86 | 4.34 | 0.4240 |
| 39 | kPa | 8.40 | 9.41 | 12.0 | 5.30 | 6.63 | 0.6072 |
| 40 | N | 0.54 | 0.76 | 41.6 | 0.36 | 0.56 | 0.8036 |
| 41 | kPa | 4.54 | 5.28 | 16.2 | 3.78 | 4.60 | 0.2101 |
| 42 | kPa | 7.99 | 9.15 | 14.5 | 5.51 | 6.75 | 0.1796 |
| 43 | kPa/s | -1.82 | -0.71 | -61.0 | 1.84 | 2.21 | 0.6072 |
| 44 | %/s | -6.36 | -2.87 | -54.9 | 6.94 | 9.32 | 0.2101 |
| 45 | kPa/s | -1.18 | -0.95 | -19.6 | 1.16 | 1.10 | 1.0000 |
| 46 | %/s | -7.11 | -4.84 | -31.8 | 5.04 | 6.60 | 0.3018 |
| 47 | N | 1.40 | 1.65 | 17.5 | 0.81 | 0.78 | 0.4545 |
| 48 | kPa | 8.23 | 8.45 | 2.7 | 4.71 | 5.05 | 0.8036 |
| 49 | mm | 8.69 | 2.47 | -71.6 | 7.24 | 4.88 | 0.0042 |
| 50 | N | 1.51 | 1.85 | 23.0 | 0.99 | 0.82 | 0.2101 |
| 51 | kPa | 8.16 | 9.49 | 16.3 | 4.46 | 4.86 | 0.4545 |
| 52 | mm | 6.05 | 4.24 | -29.9 | 5.68 | 4.61 | 0.4240 |

**Table 4. VTI Parameters for Treatment Group at Baseline and Study Completion**

| **VTI Parameter No.** | **Parameter Units** | **Average (Baseline) (N=16)** | **Average (6 (weeks) (N=16)** | **100 (Post-Pre)/Pre, %** | **PreTreatment Std. Dev.** | **PostTreatment Std. Dev.** | **P-value for Wilcoxon Signed Rank Test** |
|---|---|---|---|---|---|---|---|
| 1 | N | 0.41 | 0.54 | 33.2 | 0.21 | 0.23 | 0.0111 |
| 2 | mJ | 17.08 | 20.93 | 22.5 | 8.29 | 9.81 | 0.1189 |
| 3 | kPa/mm | 0.49 | 0.60 | 21.7 | 0.37 | 0.51 | 0.3336 |
| 4 | kPa/mm | 0.35 | 0.45 | 27.0 | 0.20 | 0.28 | 0.4212 |
| 5 | kPa | 8.02 | 10.81 | 34.8 | 4.67 | 5.34 | 0.0497 |
| 6 | kPa | 7.46 | 8.51 | 14.0 | 4.78 | 4.09 | 0.1635 |
| 7 | kPa | 5.11 | 5.39 | 5.5 | 2.71 | 2.80 | 0.8581 |
| 8 | kPa | 4.23 | 4.93 | 16.4 | 2.20 | 2.75 | 0.1470 |
| 9 | kPa | 4.90 | 4.94 | 0.8 | 3.54 | 3.74 | 0.9875 |
| 10 | kPa | 3.48 | 4.49 | 29.1 | 1.41 | 1.78 | 0.0659 |
| 11 | kPa | 4.95 | 5.43 | 9.7 | 2.38 | 1.62 | 0.1925 |
| 12 | kPa | 4.29 | 4.17 | -2.8 | 2.88 | 2.16 | 0.7734 |
| 13 | kPa/mm | 0.23 | 0.34 | 50.7 | 0.20 | 0.28 | 0.1238 |
| 14 | kPa/mm | 0.15 | 0.19 | 24.6 | 0.14 | 0.14 | 0.3656 |
| 15 | kPa/mm | 0.17 | 0.29 | 63.4 | 0.14 | 0.51 | 0.9233 |
| 16 | kPa/mm | 0.20 | 0.34 | 71.7 | 0.08 | 0.36 | 0.1152 |
| 17 | kPa/mm | 0.20 | 0.18 | -8.6 | 0.14 | 0.11 | 0.7542 |
| 18 | kPa/mm | 0.21 | 0.19 | -9.7 | 0.17 | 0.16 | 0.4294 |
| 19 | kPa | 7.07 | 10.24 | 44.9 | 1.33 | 3.48 | 0.0016 |
| 20 | N | 1.53 | 1.79 | 16.9 | 0.42 | 0.58 | 0.1923 |
| 21 | N | 0.92 | 1.08 | 17.7 | 0.43 | 0.49 | 0.2025 |
| 22 | kPa | 3.81 | 4.15 | 8.9 | 1.65 | 2.45 | 0.7020 |
| 23 | kPa | 3.45 | 4.81 | 39.4 | 1.46 | 2.65 | 0.1101 |
| 24 | kPa | 4.52 | 4.92 | 8.8 | 1.78 | 2.43 | 0.3494 |
| 25 | N | 1.22 | 1.26 | 3.2 | 0.64 | 0.47 | 0.8469 |
| 26 | kPa | 6.80 | 7.15 | 5.2 | 4.10 | 4.89 | 0.5900 |
| 27 | mm | 2.26 | 1.62 | -28.3 | 5.18 | 4.85 | 0.7720 |
| 28 | N | 1.23 | 1.25 | 1.5 | 0.65 | 0.53 | 0.5614 |
| 29 | kPa | 5.81 | 5.91 | 1.7 | 2.87 | 2.85 | 0.5708 |
| 30 | mm | 1.16 | 2.51 | 116.7 | 4.33 | 4.39 | 0.6785 |
| 31 | N | 1.00 | 1.09 | 9.6 | 0.58 | 0.72 | 0.5245 |
| 32 | kPa | 10.72 | 10.27 | -4.2 | 10.30 | 11.03 | 0.9780 |
| 33 | kPa | 14.83 | 14.70 | -0.9 | 10.86 | 12.67 | 0.6483 |
| 34 | N | 1.12 | 1.24 | 10.8 | 0.68 | 0.66 | 0.4353 |
| 35 | kPa | 7.92 | 7.98 | 0.8 | 5.57 | 4.68 | 1.0000 |
| 36 | kPa | 11.75 | 12.19 | 3.7 | 6.22 | 5.28 | 0.6788 |
| 37 | N | 0.55 | 0.78 | 43.0 | 0.49 | 0.56 | 0.0125 |
| 38 | kPa | 3.74 | 4.60 | 23.0 | 4.26 | 4.04 | 0.0835 |
| 39 | kPa | 7.39 | 7.51 | 1.5 | 6.32 | 4.96 | 0.7516 |
| 40 | N | 0.51 | 0.81 | 59.5 | 0.48 | 0.65 | 0.0203 |
| 41 | kPa | 3.83 | 5.28 | 38.0 | 4.54 | 5.34 | 0.0479 |
| 42 | kPa | 7.19 | 8.31 | 15.5 | 6.65 | 7.10 | 0.0497 |
| 43 | kPa/s | -1.66 | -0.44 | -73.4 | 2.32 | 1.00 | 0.0033 |
| 44 | %/s | -11.30 | -4.04 | -64.3 | 5.88 | 5.09 | 0.0007 |
| 45 | kPa/s | -1.03 | -0.35 | -65.6 | 0.95 | 0.43 | 0.0043 |
| 46 | %/s | -10.97 | -3.49 | -68.2 | 7.53 | 3.39 | 0.0004 |
| 47 | N | 1.95 | 1.85 | -4.9 | 0.74 | 0.79 | 0.7615 |
| 48 | kPa | 10.01 | 9.07 | -9.5 | 3.29 | 3.01 | 0.5702 |
| 49 | mm | 9.35 | 4.15 | -55.7 | 8.29 | 6.53 | 0.1311 |
| 50 | N | 2.04 | 1.98 | -3.0 | 0.78 | 0.93 | 0.9891 |
| 51 | kPa | 9.32 | 8.26 | -11.4 | 3.77 | 2.82 | 0.2348 |
| 52 | mm | 7.53 | 6.26 | -16.8 | 6.85 | 7.18 | 0.6286 |

**Table 5. Within-Group Analysis of Primary and Secondary Outcomes at Baseline and Study Completion**

| | **Control Group at Baseline (N=16)** | **Control Group at 6 Weeks (N=16)** | **P-Value** | **Treatment Group at Baseline (N=16)** | **Treatment Group at 6 weeks (N=16)** | **P-Value** |
|---|---|---|---|---|---|---|
| UDI-6 score (mean ± SD) | 43±18 | 33±26 | 0.22 | 45±21 | 29±21 | **0.02** |
| IIQ-7 score before (mean ± SD) | 48±23 | 40±28 | 0.36 | 50±30 | 30±21 | **0.01** |
| Maximum vaginal squeeze pressure (cmH₂O, mean ± SD) | 36±18 | 41±21 | 0.13 | 30±15 | 45±28 | **0.001** |
| Oxford Scale (mean ± SD) | 2.4±0.8 | 2.9±0.5 | **0.01** | 2.2±0.6 | 2.8±0.5 | **0.0005** |
| PGI-S score (mean ± SD) | 2.8±0.8 | 2.6±0.8 | 0.45 | 3.1±0.8 | 2.3±0.8 | **0.0001** |

| | | | | | | |
|---|---|---|---|---|---|---|
| Urogenital Distress Inventory (UDI-6), Incontinence Impact Questionnaire (IIQ-7), Patient Global Impression of Severity (PGI-S) | | | | | | |

**Table 6. Control Group BI-Score Measured by VTI at Baseline and Study Completion**

| | **Parameter Units** | **Baseline (N=16)** | **6 weeks (N=16)** | **P-value for Wilcoxon Signed Rank Test** |
|---|---|---|---|---|
| Elasticity | SD | -1.24 | -1.02 | 0.0787 |
| Support | SD | -0.74 | -0.70 | 0.4380 |
| **Contraction** | **SD** | **-0.87** | **-0.55** | **0.0084** |
| Relaxation | SD | -0.68 | -0.06 | 0.0703 |
| Mobility | SD | 0.23 | 0.22 | 0.3520 |
| **BI-score** | **SD** | **-0.66** | **-0.42** | **0.0494** |

**Table 7. Treatment Group BI-Score Measured by VTI at Baseline and Study Completion**

| | **Parameter Units** | **Baseline (N=16)** | **6 weeks (N=16)** | **P-value for Wilcoxon Signed Rank Test** |
|---|---|---|---|---|
| **Elasticity** | **SD** | **-1.47** | **-1.24** | **0.0353** |
| Support | SD | -1.24 | -0.97 | 0.1070 |
| Contraction | SD | -1.02 | -0.92 | 0.1876 |
| **Relaxation** | **SD** | **-1.55** | **0.09** | **0.0004** |
| Mobility | SD | -0.03 | 0.17 | 0.5995 |
| **BI-score** | **SD** | **-1.06** | **-0.58** | **0.0012** |

### Conclusions

The study demonstrated a significant improvement in the treatment group in all examined primary and secondary outcomes compared to baseline. Women with SUI receiving a specially formulated composition in addition to daily pelvic floor muscle exercise for six weeks had significantly greater change in vaginal squeeze pressure, greater improvement in BI-score, and a larger decrease in PGI-S score, compared to women in the control group. Women in the treatment group, on average, improved twice as much in their BI-score as women in the control group. In addition, women in the treatment group had improved urinary symptoms (significant decrease in UDI-6 score and IIQ-7) and stronger vaginal squeeze pressure and BI-score compared to baseline.

This is the first report showing that daily administration of a pharmaceutical composition comprising creatine, leucine, zinc, magnesium, and calcium, combined with daily PFMT, resulted in improvement in pelvic floor muscle strength and BI, which indeed led to better urinary control, after only six weeks of treatment.

Various preferred features and embodiments of the present invention will now be described with reference to the following numbered paragraphs (paras).

A pharmaceutical composition comprising 2-4 g creatine, 250-750 mg leucine, 2-8 mg zinc, 60-200 mg calcium, and 20-100 mg magnesium, wherein the composition is formulated for oral delivery.

The pharmaceutical composition of para [0078] comprising 3 g creatine, 500 mg leucine, 5 mg zinc, 120 mg calcium, and 60 mg magnesium.

The pharmaceutical composition of para [0078], wherein the creatine is creatine monohydrate.

The pharmaceutical composition of para [0078], wherein the zinc is zinc sulfate.

The pharmaceutical composition of para [0078], wherein the calcium is calcium citrate.

The pharmaceutical composition of para [0078], wherein the magnesium is magnesium citrate.

A kit comprising the pharmaceutical composition of para [0078] and instructions for administration.

The kit of para[0084], comprising instructions for pelvic floor muscle training (PFMT).

The pharmaceutical composition of para [0078] or the kit of para [0084] for use in a method of improving biomechanical integrity of the pelvic floor of a female subject.

The pharmaceutical composition of para [0078] or the kit of para [0084] for use in a method of treating or preventing urinary incontinence in a female subject.

A method of improving biomechanical integrity (BI) of the pelvic floor of a female subject undergoing pelvic floor muscle training (PFMT), the method comprising orally administering to the subject 2-4 g/day creatine, 250-750 mg/day leucine, 2-8 mg/day zinc, 60-200 mg/day calcium, and 20-100 mg/day magnesium, wherein administration continues daily for at least six weeks.

The method of para [0088] comprising orally administering to the subject 3 g/day creatine, 500 mg/day leucine, 5 mg/day zinc, 120 mg/day calcium, and 60 mg/day magnesium.

The method of para [0088], wherein the creatine is creatine monohydrate.

The method of para [0088], wherein the zinc is zinc sulfate.

The method of para [0088], wherein the calcium is calcium citrate.

The method of para [0088], wherein the magnesium is magnesium citrate.

The method of para [0088], wherein administration is once daily.

The method of para [0088], wherein the PFMT comprises 30-60 pelvic floor muscle contractions per day.

The method of para [0088], wherein the subject's BI score is improved by at least 0.40 (SD) compared to the subject's baseline BI score.

The method of para [0088], wherein the subject's BI score is improved by at least 40% compared to the subject's baseline BI score.

A method of treating urinary incontinence in a female subject undergoing pelvic floor muscle training (PFMT), the method comprising orally administering to the subject 2-4 g/day creatine, 250-750 mg/day leucine, 2-8 mg/day zinc, 60-200 mg/day calcium, and 20-100 mg/day magnesium, wherein administration continues daily for at least six weeks.

The method of para [0098] comprising orally administering to the subject 3 g/day creatine, 500 mg/day leucine, 5 mg/day zinc, 120 mg/day calcium, and 60 mg/day magnesium.

The method of para [0098], wherein the creatine is creatine monohydrate.

The method of para [0098], wherein the zinc is zinc sulfate.

The method of para [0098], wherein the calcium is calcium citrate.

The method of para [0098], wherein the magnesium is magnesium citrate.

The method of para [0098], wherein administration is once daily.

The method of para [0098], wherein the PFMT comprises 30-60 pelvic floor muscle contractions per day.

A method of preventing urinary incontinence (UI) in a female subject undergoing pelvic floor muscle training (PFMT), the method comprising orally administering to the subject 2-4 g/day creatine, 250-750 mg/day leucine, 2-8 mg/day zinc, 60-200 mg/day calcium, and 20-100 mg/day magnesium, wherein the subject is at-risk for developing UI, and wherein administration continues daily for at least six weeks.

The method of para [0106] comprising orally administering to the subject 3 g/day creatine, 500 mg/day leucine, 5 mg/day zinc, 120 mg/day calcium, and 60 mg/day magnesium.

The method of para [0106], wherein the creatine is creatine monohydrate.

The method of para [0106], wherein the zinc is zinc sulfate.

The method of para [0106], wherein the calcium is calcium citrate.

The method of para [0106], wherein the magnesium is magnesium citrate.

The method of para [0106], wherein administration is once daily.

The method of para [0106], wherein the PFMT comprises 30-60 pelvic floor muscle contractions per day.

The method of para [0106], wherein the subject is elderly, is obese, has undergone pelvic surgery, has undergone radiation treatment in her pelvic area, has sustained traumatic injury to her pelvic area, or has sustained nerve damage in her pelvic area.

### REFERENCES

1. Bo K, Frawley HC, Haylen BT, et al. An International Urogynecological Association (IUGA)/International Continence Society (ICS) joint report on the terminology for the conservative and nonpharmacological management of female pelvic floor dysfunction. Int Urogynecol J. Feb 2017;28(2):191-213. doi:10.1007/s00192-016-3123-4
2. Myers DL. Female mixed urinary incontinence: a clinical review. JAMA. May 21 2014;311(19):2007-14. doi:10.1001/jama.2014.4299
3. Kinchen KS, Lee J, Fireman B, Hunkeler E, Nehemiah JL, Curtice TG. The prevalence, burden, and treatment of urinary incontinence among women in a managed care plan. J Womens Health (Larchmt). Apr 2007;16(3):415-22. doi:10.1089/jwh.2006.0122
4. Morrison A, Levy R. Fraction of nursing home admissions attributable to urinary incontinence. Value Health. 2006 Jul-Aug 2006;9(4):272-4. doi:10.1111/j.1524-4733.2006.00109.x
5. Nambiar AK, Arlandis S, Bø K, et al. European Association of Urology Guidelines on the Diagnosis and Management of Female Non-neurogenic Lower Urinary Tract Symptoms. Part 1: Diagnostics, Overactive Bladder, Stress Urinary Incontinence, and Mixed Urinary Incontinence. Eur Urol. Jul 2022;82(1):49-59. doi:10.1016/j.eururo.2022.01.045
6. ACOG Practice Bulletin No. 155: Urinary Incontinence in Women. Obstet Gynecol. Nov 2015;126(5):e66-e81. doi: 10.1097/AOG.0000000000001148
7. Newman DK, Borello-France D, Sung VW. Structured behavioral treatment research protocol for women with mixed urinary incontinence and overactive bladder symptoms. Neurourol Urodyn. 01 2018;37(1):14-26. doi:10.1002/nau.23244
8. Sung VW, Borello-France D, Dunivan G, et al. Methods for a multicenter randomized trial for mixed urinary incontinence: rationale and patient-centeredness of the ESTEEM trial. Int Urogynecol J. Oct 2016;27(10):1479-90. doi:10.1007/s00192-016-3031-7
9. Garber CE, Blissmer B, Deschenes MR, et al. American College of Sports Medicine position stand. Quantity and quality of exercise for developing and maintaining cardiorespiratory, musculoskeletal, and neuromotor fitness in apparently healthy adults: guidance for prescribing exercise. Med Sci Sports Exerc. Jul 2011;43(7):1334-59. doi:10.1249/MSS.0b013e318213fefb
10. Borello-France D, Burgio KL, Goode PS, et al. Adherence to behavioral interventions for urge incontinence when combined with drug therapy: adherence rates, barriers, and predictors. Phys Ther. Oct 2010;90(10): 1493-505. doi:10.2522/ptj.20080387
11. Burgio KL, Kraus SR, Menefee S, et al. Behavioral therapy to enable women with urge incontinence to discontinue drug treatment: a randomized trial. Ann Intern Med. Aug 05 2008;149(3):161-9. doi:10.7326/0003-4819-149-3-200808050-00005
12. Dumoulin C, Cacciari LP, Hay-Smith EJC. Pelvic floor muscle training versus no treatment, or inactive control treatments, for urinary incontinence in women. Cochrane Database Syst Rev. 10 04 2018;10:CD005654. doi:10.1002/14651858.CD005654.pub4
13. Harris RC, Söderlund K, Hultman E. Elevation of creatine in resting and exercised muscle of normal subjects by creatine supplementation. Clin Sci (Lond). Sep 1992;83(3):367-74. doi:10.1042/cs0830367
14. Hultman E, Söderlund K, Timmons JA, Cederblad G, Greenhaff PL. Muscle creatine loading in men. J Appl Physiol (1985). Jul 1996;81(1):232-7. doi:10.1152/jappl. 1996.81.1.232
15. Balsom PD, Söderlund K, Ekblom B. Creatine in humans with special reference to creatine supplementation. Sports Med. Oct 1994;18(4):268-80. doi:10.2165/00007256-199418040-00005
16. Brosnan ME, Brosnan JT. The role of dietary creatine. Amino Acids. 08 2016;48(8):1785-91. doi:10.1007/s00726-016-2188-1
17. Kreider RB, Kalman DS, Antonio J, et al. International Society of Sports Nutrition position stand: safety and efficacy of creatine supplementation in exercise, sport, and medicine. J Int Soc Sports Nutr. 2017;14:18. doi:10.1186/s12970-017-0173-z
18. European Food Safety Authority. Scientific Opinion on the substantiation of health claims related to creatine and increase in physical performance during short-term, high intensity, repeated exercise bouts (ID 739, 1520, 1521, 1522, 1523, 1525, 1526, 1531, 1532, 1533, 1534, 1922, 1923, 1924), increase in endurance capacity (ID 1527, 1535), and increase in endurance performance (ID 1521, 1963) pursuant to Article 13(1) of Regulation (EC) No 1924/20061. EFSA Journal 2011;9(7):2303
19. Trabal J, Forga M, Leyes P, et al. Effects of free leucine supplementation and resistance training on muscle strength and functional status in older adults: a randomized controlled trial. Clin Interv Aging. 2015;10:713-23. doi:10.2147/CIA.S75271
20. Rowlands DS, Nelson AR, Phillips SM, et al. Protein-leucine fed dose effects on muscle protein synthesis after endurance exercise. Med Sci Sports Exerc. Mar 2015;47(3):547-55. doi:10.1249/MSS.0000000000000447
21. Luiking YC, Deutz NE, Memelink RG, Verlaan S, Wolfe RR. Postprandial muscle protein synthesis is higher after a high whey protein, leucine-enriched supplement than after a dairy-like product in healthy older people: a randomized controlled trial. Nutr J. Jan 2014;13:9. doi:10.1186/1475-2891-13-9
22. Takacs P, Kozma B, Lamp6 R, Sipos A, Poka R. Randomized controlled trial for improved recovery of the pelvic floor after vaginal delivery with a specially formulated postpartum supplement. Obstet Gynecol Sci. May 2020;63(3):305-314.
   doi:10.5468/ogs.2020.63.3.305
23. Diokno AC, Brock BM, Brown MB, Herzog AR. Prevalence of urinary incontinence and other urological symptoms in the noninstitutionalized elderly. J Urol. Nov 1986;136(5):1022-5.
24. Diokno AC, Catipay JR, Steinert BW. Office assessment of patient outcome of pharmacologic therapy for urge incontinence. Int Urogynecol J Pelvic Floor Dysfunct. 2002;13(5):334-8. doi:10.1007/s001920200073
25. Afshari P, Dabagh F, Iravani M, Abedi P. Comparison of pelvic floor muscle strength in nulliparous women and those with normal vaginal delivery and cesarean section. Int Urogynecol J. Aug 2017;28(8):1171-1175. doi:10.1007/s00192-016-3239-6
26. Myer ENB, Roem JL, Lovejoy DA, Abernethy MG, Blomquist JL, Handa VL. Longitudinal changes in pelvic floor muscle strength among parous women. Am J Obstet Gynecol. Jun 2018;doi:10.1016/j.ajog.2018.06.003
27. Egorov V, van Raalte H, Takacs P, Shobeiri SA, Lucente V, Hoyte L. Biomechanical integrity score of the female pelvic floor. Int Urogynecol J. 06 2022;33(6):1617-1631. doi:10.1007/s00192-022-05120-w
28. Shumaker SA, Wyman JF, Uebersax JS, McClish D, Fantl JA. Health-related quality of life measures for women with urinary incontinence: the Incontinence Impact Questionnaire and the Urogenital Distress Inventory. Continence Program in Women (CPW) Research Group. Qual Life Res. Oct 1994;3(5):291-306. doi:10.1007BF00451721
29. Uebersax JS, Wyman JF, Shumaker SA, McClish DK, Fantl JA. Short forms to assess life quality and symptom distress for urinary incontinence in women: the Incontinence Impact Questionnaire and the Urogenital Distress Inventory. Continence Program for Women Research Group. Neurourol Urodyn. 1995;14(2):131-9. doi:10.1002/nau.1930140206
30. Yalcin I, Bump RC. Validation of two global impression questionnaires for incontinence. Am J Obstet Gynecol. Jul 2003;189(1):98-101. doi:10.1067/mob.2003.379
31. Rosenblatt P, McKinney J, Rosenberg RA, Iglesias RJ, Sutherland RC, Pulliam SJ. Evaluation of an accelerometer-based digital health system for the treatment of female urinary incontinence: A pilot study. Neurourol Urodyn. 09 2019;38(7):1944-1952. doi:10.1002/nau.24097
32. Faul F, Erdfelder E, Lang AG, Buchner A. G*Power 3: a flexible statistical power analysis program for the social, behavioral, and biomedical sciences. Behav Res Methods. May 2007;39(2):175-91. doi:10.3758/bf03193146
33. Barber MD, Spino C, Janz NK, et al. The minimum important differences for the urinary scales of the Pelvic Floor Distress Inventory and Pelvic Floor Impact Questionnaire. Am J Obstet Gynecol. May 2009;200(5):580.e1-7. doi:10.1016/j.ajog.2009.02.007
34. Pereira VS, Hirakawa HS, Oliveira AB, Driusso P. Relationship among vaginal palpation, vaginal squeeze pressure, electromyographic and ultrasonographic variables of female pelvic floor muscles. Braz J Phys Ther. 2014 Sep-Oct 2014;18(5):428-34.
35. Hirakawa T, Suzuki S, Kato K, Gotoh M, Yoshikawa Y. Randomized controlled trial of pelvic floor muscle training with or without biofeedback for urinary incontinence. Int Urogynecol J. Aug 2013;24(8):1347-54. doi:10.1007/s00192-012-2012-8
36. Kim EY, Kim SY, Oh DW. Pelvic floor muscle exercises utilizing trunk stabilization for treating postpartum urinary incontinence: randomized controlled pilot trial of supervised versus unsupervised training. Clin Rehabil. Feb 2012;26(2):132-41.
   doi:10.1177/0269215511411498
37. Candow DG, Chilibeck PD, Forbes SC. Creatine supplementation and aging musculoskeletal health. Endocrine. Apr 2014;45(3):354-61. doi:10.1007/s12020-013-0070-4
38. Chasapis CT, Ntoupa PA, Spiliopoulou CA, Stefanidou ME. Recent aspects of the effects of zinc on human health. Arch Toxicol. 05 2020;94(5): 1443-1460. doi:10.1007/s00204-020-02702-9
39. Csik6s A, Kozma B, Baranyai E, et al. Evaluation of zinc and copper levels in vaginal tissues and whole blood: correlation with age. BMC Womens Health. 02 11 2021;21(1):62. doi:10.1186/s12905-021-01215-6
40. Sipos AG, Pákozdy K, Jäger S, Larson K, Takacs P, Kozma B. Fractional CO. BMC Womens Health. 06 06 2021;21(1):235. doi:10.1186/s12905-021-01379-1
41. Córdova A, Caballero-Garcia A, Noriega-González D, Bello HJ, Pons A, Roche E. Nitric-Oxide-Inducing Factors on Vitamin D Changes in Older People Susceptible to Suffer from Sarcopenia. Int J Environ Res Public Health. 05 13 2022;19(10)doi:10.3390/ijerph19105938
42. Veronese N, Berton L, Carraro S, et al. Effect of oral magnesium supplementation on physical performance in healthy elderly women involved in a weekly exercise program: a randomized controlled trial. Am J Clin Nutr. Sep 2014;100(3):974-81.
   doi:10.3945/ajcn.113.080168

The foregoing description of the specific embodiments will so fully reveal the general nature of the invention that others can, by applying knowledge within the skill of the art, readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance. The present invention is further described by the following claims.

## Claims

1. A pharmaceutical composition comprising 2-4 g creatine, 250-750 mg leucine, 2-8 mg zinc, 60-200 mg calcium, and 20-100 mg magnesium, wherein the composition is formulated for oral delivery.

2. The pharmaceutical composition of claim 1 comprising 3 g creatine, 500 mg leucine, 5 mg zinc, 120 mg calcium, and 60 mg magnesium.

3. The pharmaceutical composition of claim 1 or 2, wherein the creatine is creatine monohydrate.

4. The pharmaceutical composition of any one of claims 1 to 3, wherein the zinc is zinc sulfate.

5. The pharmaceutical composition of any one of claims 1 to 4, wherein the calcium is calcium citrate.

6. The pharmaceutical composition of any one of claims 1 to 5, wherein the magnesium is magnesium citrate.

7. The pharmaceutical composition of any one of claims 1 to 6, comprising 2-4 g creatine monohydrate, 250-750 mg leucine, 2-8 mg zinc sulfate, 60-200 mg calcium citrate, and 20-100 mg magnesium citrate.

8. A kit comprising the pharmaceutical composition of any one of claims 1 to 7 and instructions for administration, optionally comprising instructions for pelvic floor muscle training (PFMT).

9. The pharmaceutical composition of any one of claims 1 to 7 for use in a method of improving biomechanical integrity of the pelvic floor of a female subject.

10. The pharmaceutical composition of any one of claims 1 to 7 for use in a method of treating or preventing urinary incontinence in a female subject.

11. The pharmaceutical composition for use of claim 9 or 10 wherein the subject is undergoing pelvic floor muscle training (PFMT), and wherein the pharmaceutical composition is administered to the female subject daily for at least six weeks, optionally wherein the PFMT comprises 30-60 pelvic floor muscle contractions per day.

12. The pharmaceutical composition for use of any one of claims 9 to 11 wherein the pharmaceutical composition is administered once daily.

13. The pharmaceutical composition for use of any one of claims 9 to 12, wherein the subject's BI score is improved by at least 0.40 (SD) compared to the subject's baseline BI score, optionally wherein the subject's BI score is improved by at least 40% compared to the subject's baseline BI score.

14. The pharmaceutical composition for use of any one of claims 9 to 13, wherein the subject is at-risk of developing UI.

15. The pharmaceutical composition for use of any one of claims 9 to 14 wherein the subject is elderly, is obese, has undergone pelvic surgery, has undergone radiation treatment in her pelvic area, has sustained traumatic injury to her pelvic area, or has sustained nerve damage in her pelvic area.
